(19) **Europäisches Patentamt / European Patent Office / Office européen des brevets**

(11) **EP 3 010 556 B1**

(12) **EUROPEAN PATENT SPECIFICATION**

(45) Date of publication and mention
of the grant of the patent:
**30.01.2019 Bulletin 2019/05**

(51) Int Cl.:
**A61L 27/48** *(2006.01)* **C12N 5/071** *(2010.01)*
**A61L 27/26** *(2006.01)* **A61L 27/38** *(2006.01)*
**A61L 27/56** *(2006.01)* **A61L 27/58** *(2006.01)*

(21) Application number: **14731159.1**

(22) Date of filing: **16.06.2014**

(86) International application number:
**PCT/EP2014/001626**

(87) International publication number:
**WO 2014/202199 (24.12.2014 Gazette 2014/52)**

(54) **MATRIX AND IMPLANT FOR TISSUE ENGINEERING**

MATRIX UND IMPLANTAT ZUR GEWEBEZÜCHTUNG

MATRICE ET IMPLANT POUR L'INGÉNIERIE TISSULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2013 EP 13003086**

(43) Date of publication of application:
**27.04.2016 Bulletin 2016/17**

(73) Proprietor: **Baer, Hans U.**
**8807 Freienbach (CH)**

(72) Inventor: **Baer, Hans U.**
**8807 Freienbach (CH)**

(74) Representative: **Schaad, Balass, Menzl & Partner AG**
**Dufourstrasse 101**
**Postfach**
**8034 Zürich (CH)**

(56) References cited:
**EP-A1- 2 256 155     EP-B1- 2 342 317**

• **G Chen ET AL: "A biodegradable hybrid sponge nested with collagen microsponges", Journal of Biomedical Materials Research, 1 August 2000 (2000-08-01), pages 273-279, XP055090692, UNITED STATES DOI: 10.1002/(SICI)1097-4636(200008)51:2<273::A ID-JBM16>3.0.CO;2-O Retrieved from the Internet: URL:http://www.ncbi.nlm.nih.gov/pubmed/108 25227 [retrieved on 2013-11-28]**
• **NAOKI KAWAZOE ET AL: "cell leakproof PLGA-collagen hybrid scaffold for cartilage tissue engineering", BIOTECHNOLOGY PROGRESS, AMERICAN INSTITUTE OF CHEMICAL ENGINEERS, US, vol. 26, no. 3, 1 May 2010 (2010-05-01), pages 819-826, XP002658848, ISSN: 8756-7938, DOI: 10.1002/BTPR.375 [retrieved on 2009-12-28]**

**Description**

The present invention relates to an implant for tissue engineering comprising a matrix seeded with at least one cell of Islets of Langerhans according to claim 1 and to a method for producing said implant according to claim 12.

Tissue engineering is an interdisciplinary field which combines engineering and material sciences with medicine and provides an alternative approach in the treatment of malfunctioning or lost organs. In this approach, temporary scaffolds (also called matrices) serve as an adhesive substrate for the implanted cells and a physical support to guide the formation of the new organs. Such matrices are therefore utilized to guide the process of tissue development by delivering cells to desired sites in the body, defining a potential space for engineered tissue, and promoting cell growth. In order to enable these processes, matrices for tissue engineering should meet the following criteria:
The surface should permit cell adhesion, promote cell growth, and allow the retention of differentiated cell functions. Further, the matrices should be biocompatible and biodegradable such that the matrices can eventually be eliminated *in vivo* without causing inflammation or toxic degradation by-products. With regard to their structure, the porosity of the matrices should be high enough to provide sufficient space for cell adhesion, extracellular matrix regeneration, and minimal diffusional constraints during culture. Their pore structure should allow even spatial cell distribution throughout the matrix to facilitate homogeneous tissue formation and the material should be reproducibly processable into three-dimensional structure, as well as mechanically strong.

A number of three-dimensional porous matrices fabricated from various kinds of biodegradable materials have been developed. EP-A-2256155, for instance, discloses porous matrices based on a biologically compatible polymer or polymer mixture. According to this document, the porous matrices are prepared by a so-called leaching procedure in which a mixture composed of polymer particles and salt particles having a defined grain size is compacted and the salt particles are subsequently leached out.

Nowadays, use is generally made of synthetic biodegradable polymers since they are easily formable into desired shapes and have better mechanical strengths than naturally derived polymers. Synthetic biodegradable polymers have the additional advantage that their periods of degradation can also be manipulated by controlling the crystallinity, molecular weight, and copolymer ratio.

Hybrid matrices made from different types of polymers have also been investigated. For instance, a scientific publication of G. Chen et al.: "A biodegradable hybrid sponge nested with collagen microsponges", Journal of Biomedical Materials Research, 2000-08-01, pages 273-279. discloses a matrix for tissue engineering comprising biodegradable hybrid sponges of porous poly(DL-lactic-co-glycolic acid) (PLGA) with collagen microsponges in the pores of the PLGA matrix. The porous PLGA matrix is obtained by a dispersing 355-425 $\mu$m sodium chloride particles in a solution of PLGA in chloroform, evaporating the solvent and leaching the salt.

Further, a scientific publication of Naoki Kawazoe et al.: "cell leakproof PLGA-collagen hybrid scaffold for cartilage tissue engineering", Biotechnology Process, American Institute of Chemical Engineers, vol. 26, no. 3, 2010-05-01, pages 819-826, discloses a cell leakproof porous PLGA-collagen hybrid scaffold prepared by wrapping the surfaces of a collagen sponge except the top surface for cell seeding with a bi-layered PLGA mesh. Despite their advantages, matrices derived from synthetic polymers also suffer from several limitations such as the lack of cell-recognition signals, limited biological acceptance and functional capacity. A further limitation is that synthetic polymers are generally hydrophobic, as indicated by high contact angles with water, which has been found to have a strongly negative impact on cell attachment and cell seeding activities on the matrices. It is therefore an object of the present invention to provide a biocompatible and biodegradable polymer matrix, which allows for efficient cell retention and proliferation while maintaining the desirable mechanical properties of known matrices, in particular synthetic matrices. According to a more specific aspect, the present invention aims at providing a polymer matrix that allows for being used in the preparation of an implant, in particular a liver implant.

This object is achieved by the porous matrix according to claim 1, the process for preparing the matrix according to claim 12. Further preferred embodiments are subject of the dependent claims. The porous matrix for tissue engineering according to the present invention comprises a first biodegradable and biocompatible polymer component forming a three-dimensional primary structure with primary pores and further comprises a second biodegradable and biocompatible polymer component other than the first polymer component, which is selected from the group consisting of collagens, laminin, fibronectin and mixtures thereof.

The term "matrix", as used throughout this application, refers to a three-dimensional support, i.e. a scaffold- or sponge-like structure, which is suitable for being colonized by cells. In this sense, the matrix serves as a three-dimensional template which can be colonized by cells or tissue. This colonization can take place *in vitro* or *in vivo*. Furthermore, the matrix serves, in connection with transplantations, for locating the transplant and also as a place holder for tissue which is gradually formed *in vivo*.

The term "biocompatible polymers" refers to polymers which are biologically tolerated and do not cause rejection when brought into a living organism. For the present invention, biocompatible polymers also encompass polymers which are recognized by a host as being foreign but whose rejection can be suppressed by appropriate immunosuppression.

The expression "biodegradable" refers to a material which can be converted into metabolizable products in living organisms (or body fluids or cell cultures derived from living organisms). Biologically degradable polymers include, for example, polymers which are bioresorbable and/or bioerodable. "Bioerodable" denotes the ability to be soluble or suspendable in biological liquids. Bioresorbable means the ability to be able to be taken up by cells, tissues or fluids of a living organism.

The first polymer component can thus in principle be any biodegradable and biocompatible polymer which can be used in the field of medicine and, in particular, in transplantation medicine. As will be described in detail below, the first polymer compound is generally a synthetic polymer compound. Compared to natural polymers, synthetic polymers generally provide a higher mechanical strength and therefore allow for preparing a matrix which is suitable to serve as a physical support for cell attachment and colonization.

It has now surprisingly been found that if a second polymer component is present forming a three-dimensional secondary structure with secondary pores, the secondary structure being contained within the interior space of at least a part of the primary pores of the primary structure, the hydrophilic properties of the matrix are significantly enhanced, while the beneficial properties, i.e. the mechanical strength of the first biodegradable and biocompatible polymer component can be maintained. Thanks to the enhanced hydrophilic properties, cell attachment and cell colonisation on the matrix is greatly facilitated. This in turn increases the chance of a successful implantation procedure, i.e. that an implanted matrix is accepted by the recipient and no rejection occurs.

The present invention therefore provides a solution that allows for combining the beneficial properties of both, synthetic and natural polymers.

In particular, the present invention allows for using a synthetic polymer as first biodegradable and biocompatible polymer component that provides the matrix with the necessary mechanical strength, whereas the second biodegradable and biocompatible polymer component being a natural polymer selected from the group of collagen, laminin and fibronectin provides the matrix with greatly beneficial hydrophilic properties. Hydrophilicity of a polymer matrix for use in tissue engineering is considered to be very important for homogeneous and sufficient cell seeding in three dimensions.

Furthermore, as will be explained in more detail below, the presence of the second polymer component highly increases the number of possible design options with regard to the pore structure of the inventive matrix. Depending on the specific arrangement of the second polymer component within pores of the primary polymer component, the size and form of the primary pores can be freely adapted. This allows, for instance, for embedding and seeding of more than one cell type within pores of the matrix by providing both, larger pores for accommodating cells of larger cell size as well as smaller pores for retaining cells of smaller cell size.

The word "pores" is used to designate the cavities or void regions which are present in the matrix according to the invention. They may have a round shape and/or an angular, in particular octagonal, shape in a 2-dimensional section and/or a canted shape when seen 3-dimensionally. The shape is furthermore preferably characterized by extensions such that the shape of the cavities can be compared with the shape of nerve cells. As such, the term "pores" also refers to cavities formed by filaments enclosing a void region. These pores or cavities may also be interconnected, meaning that the pore walls between two adjacent pores can comprise holes, forming a connection between said adjacent pores. Although both, the "primary pores" and the "secondary pores" are referred to as "pores", they may be structurally distinct from one another, e.g. with regard to their shape, size and/or interconnectivity.

The size of a pore can be specified by means of its average pore diameter - that is the mean of the longest and shortest diameters of the pores which can be discerned in a two-dimensional section. Pore size and pore distribution can be determined by means of scanning electron microscope (SEM), for instance, in a manner well known to the skilled person.

The matrix of the present invention has the further advantage that it not only allows for adapting the size of the pores therein, but it is further possible to prepare the matrix with variable permeabilities and/or porosities by adapting the concentration gradients of pores having a given size and/or shape.

For instance, the pores may be either highly interconnected or highly disjoint. Highly interconnected pores generally yield composition with increased permeability (such as hydraulic permeability or permeability to a given class of materials), while highly disjoint pores generally yield compositions with reduced permeability for the same porosity, which is calculated by dividing the void volume by the total volume of a representative sample of the matrix.

Thus, if the matrix should perform the function of a permeable or semi-permeable matrix with increased permeability, the matrix will be designed with a higher number of interconnected pores. On the other hand, a structure with a higher number of disjoint pores may be advantageous for matrices that need to have particularly high mechanical stability.

According to the present invention, the primary structure of the matrix allows for the diffusion of liquids and (macro-)molecules into the matrix or even pass through the matrix. For its use in tissue engineering, this permeability, obeying Fick's equation, is a great advantage of the inventive matrix as the exchange of gases, e.g. oxygen, liquids and compounds, such as cells nutrients and waste, is essential for allowing and promoting cell proliferation in and close to such matrices.

In addition, it has been found that the surface of the primary structure of the matrix is able to activate growth factors, such as vessel growth factors (VGF), by interaction with tissue of the recipient at the site of implantation. The activated growth factors are important to stimulate and attract cells that lead to the formation and ingrowth of small vessels (capillaries) into the matrix. The growth factors that are activated may already be present in the surrounding tissue at the implantation site or may also be provided to the implantation site with the matrix, e.g. as component in the primary and/or secondary polymer material, inside the pores of the primary and/or secondary structure, or by the matrix being coated with a material comprising the growth factors.

The matrix according to the present invention can therefore perfectly be adjusted to the specific function of the organ to be repaired or replaced by the matrix. In addition, due to its enhanced hydrophilic properties, the inventive matrix meets highly improved acceptance by a recipient and is therefore outstandingly well suited for use in tissue engineering.

With regard to the material of the matrix, it is further preferred that the first polymer component is selected from the group consisting of poly(glycolic acid) (PGA), poly(lactic acid)(PLA), poly(glycolic acid-lactic acid) (PGLA) and mixtures thereof. Polymer components of lactic acid (PLA), for example, poly-L-lactic acid (PLLA), poly-D,L-lactic acid (racemic mixture of L- and D-lactides; PDLLA), poly-glycolic acid (PGA) or mixtures thereof (PLGA; now also termed poly (lactide-co-glycolide) or PLG) are attractive candidates for fabricating biodegradable matrices due to their flexible and well defined physical properties and relative biocompatibility. Additionally, their degradation products are low molecular weight compounds, such as lactic acid and glycolic acid, which enter into normal metabolic pathways. Furthermore, copolymers of poly(lactic-co-glycolic acid) offer the advantage of a large spectrum of degradation rates from a few days to years by simply varying the copolymer ratio of lactic acid to glycolic acid.

In a particularly preferred embodiment, the first polymer is a copolymer of PGA and PLA. The properties of these polymers can be tuned by changing the polymer composition within the basic PLA/PGA theme.

More specifically, the first polymer component is preferably poly(glycolic acid-lactic acid) having a lactic acid content of about 85 mol% and a glycolic acid content of about 15 mol%. Such a mixture of poly(lactid acid) (PLLA) and poly (lactide-co-glycolide) (85/15) (PLGA 85/15) can be purchased, for instance, from Evonik Industries AG (Essen, Germany). Further preferred PGA/PLA mixtures are poly(D,L-lactide-co-glycolide) 50:50, e.g. RESOMER® RG 502; poly(D,L-lactide-co-glycolide) 65:35, e.g. RESOMER® RG 653; poly(D,L-lactide-co-glycolide) 75:25, e.g. RESOMER® RG 752; poly(D,L-lactide-co-glycolide) 85:15, e.g. RESOMER® RG 858.

As mentioned above, the second polymer component is selected from the group consisting of collagens, laminin, fibronectin and mixtures thereof, which are extracellular matrix proteins which can be prepared in purified form in a manner known per se or else obtained commercially.

From these extracellular matrix proteins, collagen is most preferred. Thanks to its nano-fibrous architecture, collagen is particularly effective in promoting cell adhesion, growth and differentiated function in tissue cultures. However, it has also been found that if the second polymer component comprises collagen, the hydrophilic properties of the matrix according to the present invention are particularly enhanced. In this regard, the term "collagen" as used in the context of the present invention encompasses naturally derived collagens and synthetically produced collagens and also collagen derived substances, such as gelatine, which is a hydrolysed form of collagen.

The matrix of the present invention therefore provides a hybrid structure of two biodegradable polymer components which is stable, can be prepared in different shapes, and further allows for good cell interaction and hydrophilicity.

Based on the total weight of the matrix, the weight ratio of primary structure to secondary structure is preferably in the range of about 1 to 100 %.

It is further particularly preferred that the second polymer component essentially consists of collagen. In this regard, the second polymer component may consist of only one type of collagen, i.e. type I, or may consist of a mixture of collagen types, i.e. a mixture of type I collagen and type IV collagen. In the latter case, preference is given to the mixture containing the proteins in approximately equal percentages by weight. Collagen type I is one of the main components of natural blood vessels and provides the secondary structure with cellular attachment sites as well as tensile strength. As mentioned above, the second polymer component is contained within the primary pores of the primary pore structure that provide physical surfaces, onto which the cells can lay their own extracellular matrix three-dimensionally. Preferably, the primary pores form an interconnected pore structure comprising a channel network.

It is particularly preferred that the second polymer component is gelatine. Gelatine is a hydrolysed form of collagen, also called collagen hydrolysate. The amino acid content of hydrolysed collagen is the same as of collagen. It is commonly used as a gelling agent in food, pharmaceuticals, and cosmetic manufacturing.

In the primary structure, the primary pores have preferably an average pore size diameter of 150 $\mu$m to 300 $\mu$m, preferably 200 $\mu$m to 300 $\mu$m. A porous structure with - preferably interconnected - pores of this pore size allows for improved nutrient transport to the center of the matrix through the primary structure and also prevents the formation of large cell clusters that can potentially develop into necrotic centers due to lack of nutrition. As mentioned, pore size and pore distribution can be determined by means of scanning electron microscope (SEM), which is a standard procedure in this regard and is well known to the skilled person.

In order to even further improve the physiological functionality of the inventive matrix, the second polymer component forms a three-dimensional secondary structure with secondary pores. Thereby a three-dimensional porous secondary structure is formed within the three-dimensional porous primary structure, basically forming a so-called "scaffold in scaffold" structure. In this regard it is to be noted that the second polymer component may form fibrils, fibers or foams as three-dimensional secondary structure. However, the second polymer component may also be in form of a layer coating at least part of the inner walls of the primary pores, thus forming a three-dimensional secondary structure based on the underlying three-dimensional primary structure of the first polymer component.

The two three-dimensional structures can serve different purposes. For instance, the primary structure may mainly provide physical stability to the matrix and provide sufficient void space for accommodating cells of larger cell size, whereas the secondary structure may be designed to form smaller pores for retaining cells of smaller cell size. A matrix according to this preferred embodiment is therefore particularly well suited for embedding and seeding cells of more than one cell type.

Thus, the primary and the secondary pores preferably differ in their average pore size diameter. In particular, the secondary pores preferably have an average pore size diameter smaller than the average pore size diameter of the primary pores and being in the range from 50 $\mu$m to 290 $\mu$m, preferably 50 $\mu$m to 150 $\mu$m, more preferably 50 $\mu$m to 100 $\mu$m.

A matrix having a high porosity, in particular with a large surface/volume ratio, is highly preferred as it allows for delivery of a large number of cells and facilitates invasion of fibrovascular tissue into the matrix following implantation.

It is further highly preferred that the inventive matrix is provided with different sub-structures, i.e. portions, regions, layers, surfaces, or combinations thereof, which have different permeabilities based on different average pore sizes in a respective sub-structure. In particular, the matrix preferably comprises at least two layers, wherein the average pore size diameter of the pores in a first layer is different from the average pore size diameter of the pores in a second layer.

In particular, the matrix preferably comprises a porous base layer and a porous cell-embedding layer, the average pore size diameter of the pores in the base layer being smaller than the one of the pores in the cell-embedding layer. In a specific embodiment, the average pore size diameter of the pores in the cell-embedding layer is in the range from 300 $\mu$m to 500 $\mu$m, whereas the average pore size diameter of the pores in the base layer is in the range from 10 $\mu$m to 200 $\mu$m. Thanks to this special design of the matrix, cells of larger diameter, i.e. from 200 $\mu$m to 300 $\mu$m, can be retained and colonized in the cell-embedding layer, whereas cells of smaller diameter, i.e. from 50 $\mu$m to 200 $\mu$m, can be retained and colonized in the base layer. In other words, the base layer acts as sort of a security net for retaining cells of smaller diameter, which might otherwise "fall through" the larger pores in the cell-embedding layer. The base layer thus provides effective protection against cell leakage during cell seeding. By retaining more cells in the porous matrix, efficient tissue regeneration is greatly facilitated.

It is further preferred that the pores in the base layer have an average pore size diameter in the range from 10 $\mu$m to 100 $\mu$m. This allows that also small cells such as hepatocytes, which have an average cell diameter of 20-30 $\mu$m, can effectively be retained and cultivated in the matrix of the present invention.

When the matrix is used for cell cultivation, it is important that it provides an optimal environment that promotes cell attachment, proliferation and cell migration into the matrix. As mentioned, the presence of the secondary structure provides the matrix with enhanced hydrophilic properties, which facilitates the attachment of cells to the matrix structure. However, the abilities to attach and migrate greatly vary from one cell type to another.

In order to further facilitate the attachment of cells to the surface of the matrix, the matrix is preferably provided with a hydrophilic plasma coating by plasma treatment before cells are loaded onto the matrix. Most preferably, the matrix is provided with a thin coating by plasma deposition of a polymerized substance, preferably selected from the group of (methyl)-acrylate (MA), (methyl)-acrylate anhydride (MAA) and poly(2-hydroxyethylmethacrylate) (PHEMA).

Plasma treatment is known to the person skilled in the art. It involves an attachment of a monolayer or thin polymer film to the surface and is used for improving wettability and adhesion properties of a surface prior to further processing. More specifically, it generally involves the steps of surface cleaning, surface activation and subsequent plasma coating of the surface. For cleaning and activation, the surface is normally treated with a non-polymerizable gas, such as argon, oxygen, nitrogen or fluorine, in a vacuum system. In order to obtain a matrix with any of the above described designs, the matrix can be prepared by using 3D-printing and/or plasma technology and/or electrospinning.

The use of 3D-printing for preparing the matrix of the present invention is particularly preferred since this technology allows for making a three-dimensional solid object of virtually any shape with impeccable accuracy. With regard to the matrix of the present invention, 3D-printing not only allows for producing the primary structure and/or the secondary structure but allows further to prepare a matrix with cells embedded therein, particularly cells of two different types, more particularly hepatocytes and cells of Islet of Langerhans.

The method of electro-spinning is particularly suited to form thin fibers which are deposited on a target making up a mesh structure thereon. "Target" means a shaped body which can have different forms, e.g. a circular plate-like form. The matrix according to the present invention comprises fibers of about 1 to 100 $\mu$m diameter, preferably about 10 to 40 $\mu$m.

According to a further aspect, the present invention also provides a new fabrication method that allows for a straight-forward and economic preparation of matrices described above. Said method specifically comprises the subsequent steps of:

a) providing a mixture (M) comprising

a polymer solution (PS-I) of polymer particles of the first polymer component dissolved in a polymer solvent and particles of a porogenic leachable material, said porogenic particles being insoluble in the polymer solvent and having a grain size in the range of from about 100 $\mu$m to 400 $\mu$m;

b) compacting the mixture (M) by removal of the polymer solvent (S-I);

c) removing the porogenic leachable material, thereby yielding the three-dimensional primary structure with primary pores;

d) immersing the primary structure in a solution (PS-II) comprising the second polymer component dissolved in a solvent (S-II), and

e) removing the solvent (S-II).

The first step a) of the above method involves the dissolving of the primary polymer component, e.g. PLLA or PLGA, in a polymer solvent (S-I), e.g. chloroform or methylene chloride. The resulting polymer solution (PS-I) is then mixed with a porogenic leachable material, e.g. a water a water-soluble porogenic leachable material, such as a salt, in particular NaCl, to obtain the mixture (M). The mixing can also be effected by filling a container with the porogenic leachable material and casting the polymer solution (PS-I) onto it. Then the polymer solvent (S-I) is removed, e.g. by simple evaporation, which leads to a decrease in volume of the mixture (M), and thus, to a compacted structure, or by the application of pressure on the mixture (M). After removal of the polymer solvent (SI), the porogenic leachable is leached out, e.g. by leaching the compacted mixture (M) in water if a water-soluble porogenic leachable material is used.

The resulting porosity of the primary structure can be controlled by the amount of porogenic leachable material added, while the pore size of the primary pores is dependent on the size of the crystals of the porogenic leachable material. With 70 weight percent porogenic leachable material and above, primary pores with a high interconnectivity were achieved.

The compacting is preferably effected by the action of pressure. For this, the polymer/porogenic leachable material can be pressed in a conventional hydraulic press at a ram pressure in the range of from about 780 psi to 1450 (in the following, 1 psi = 0.069 bar), advantageously in the range of from about 840 psi to about 1230 psi, and in particular in the range from about 900 psi to 1100 psi. It has proved to be expedient to allow the pressure to act for from about 10 s to 360 s, advantageously from about 40 s to 180 s, and in particular from about 50 s to 70 s, at temperatures in the range from 18 °C to 25 °C.

The porogenic leachable material is removed, for example, by dissolving it out using water or aqueous solutions. First, the compacted mixture (matrix blank) can be soaked for from about 1 h to 80 h, advantageously from about 12 h to 62 h, and in particular from about 36 h to 60 h. Alternatively, the compacted mixture can be washed with deionized water until the weight of the dried primary structure remains unchanged.

It is furthermore advantageous for the compacted mixture to be initially stored in a $CO_2$ atmosphere prior to the porogenic leachable material being removed. Thus, for example, the compacted mixture can be gassed at a $CO_2$ pressure in the range of from about 140 psi to 1650 psi, advantageously in the range of from about 360 psi to 1120 psi, and, in particular, in the range of from about 800 psi to 900 psi, with times in the range of from about 1 h to 180 h, advantageously in the range of from about 3 h to 60 h, and, in particular, in the range of from about 12 h to 36 h, having proved to be expedient in this connection. After that, the pressure is reduced, with the rate at which the pressure is lowered having an influence on the pore formation. Although the use of $CO_2$ is preferred, other gases, such as air, nitrogen, helium, neon, krypton, argon, xenon or oxygen can likewise be suitable. Subsequently, the water or the aqueous solution is, with a view to drying, removed in a manner known per se. To achieve this, the matrix can, for example, be laid on absorbent paper.

Preferably, the mixture (M) in step a) is provided by adding the polymer solution (PS-I) to a particle mixture ($M_P$) composed of polymer particles of the first polymer component and particles of the porogenic leachable material.. In other words, the mixture (M) provided in step a) of the inventive method preferably contains

i) the polymer solution (PS-I) comprising particles of the first polymer component dissolved in the polymer solvent (S-I) and

ii) a particle mixture ($M_P$) of polymer particles of the first polymer component, preferably having a grain size in the range of from about 100 $\mu$m to 300 $\mu$m, and particles of the porogenic leachable material.

In this connection, it is to be noted that both, the polymer particles in the particle mixture (M$_P$) and the polymer solution (PS-I) are/comprise polymer particles of the first polymer component - once in the dissolved state in the polymer solution and once in solid form. The amount of polymer solvent (S-I) in the polymer solution (PS-I) is preferably such that the solid polymer particles in the particle mixture (M$_P$) are not instantly dissolved when the polymer solution (PS-I) is mixed with the particle mixture (M$_P$) for preparation of the mixture (M). This way, admixing the polymer solution (PS-I) and the particle mixture (M$_P$) initially results in a stirrable paste which then rapidly becomes solid as the solvent is removed ($\rightarrow$ step b)).

In a preferred embodiment, the polymer particles of the first polymer component used for preparing the polymer solution (PS-I) by dissolving the polymer particles in a polymer solvent (S-I) have a grain size in the range of from about 100 $\mu$m to 300 $\mu$m. Particles of this size are quickly dissolved in a minimal amount of polymer solvent.

Again, the concentration of the first polymer component in the polymer solution (PS-I) is expediently to be chosen such that the first polymer component is completely dissolved, on the one hand, and, on the other hand, the solvent can be removed rapidly without the polymer particles in the particle mixture (M$_P$) beginning to dissolve to any significant extent. A weight ratio of polymer particles to dissolved polymer of from 10:1 to 1:100, advantageously of from 2:1 to 1:25, and in particular of from 1:1 to 1:10, has proved to be beneficial. As far as the weight ratio of polymer particles of the first polymer component to porogenic particles in the particle mixture (M$_P$) is concerned, it is possible, within the context of this embodiment, to select a weight ratio which is higher with respect to porogenic leachable material, i.e. of up to 1:200, 1:500 or 1:1000, with the weight ratio of total first polymer component to porogenic leachable material still being greater than 1:100. In this way, it is possible to obtain porosities of greater than 98%.

Step b) of the above method pertains to the compacting of the mixture (M) by removal of the polymer solvent (S-I). As mentioned above, the compacting is preferably effected by the action of pressure, e.g. in a conventional hydraulic press. By the action of pressure, the solvent molecules of the polymer solvent (S-I) are squeezed out of the interspace between the solid particles, in analogy to water absorbed in a sponge that is wrung out. However, the polymer solvent (S-I) may also be at least partly removed, e.g. under reduced pressure, which also leads to a compaction of the mixture (M) as the removal of solvent molecules from the interspace between the solid particles enables the molecules remaining in the mixture to come in closer proximity to each other. As a result, the volume of the mixture will decrease by removal of the polymer solvent (S-I).

The polymer solvent (S-I) which is used for preparing the polymer solution (PS-) should dissolve the first polymer component but not the porogenic leachable material. This ensures that the porogenic properties of the leachable material are not, or are only insignificantly, affected. For this purpose, acetone, ethyl acetate, methylene chloride, chloroform, hexafluoroisopropanol, chlorinated and fluorinated, aliphatic and aromatic hydrocarbons, tetrahydrofuran, ethyl methyl ketone, diethyl ketone, and mixtures thereof, are, for example, suitable for dissolving the abovementioned polymers. Chloroform is, in particular, suitable for dissolving poly(glycolic acid), poly(lactic acid) or poly(glycolic acid-lactic acid), and also suitable with a view to the medical use.

In the above-described method, the porogenic leachable material serves as a material which, by definition, is understood as being a solid, or at least semisolid, material which initially unites with the matrix-forming polymer to give a mixture and which is then removed from the mixture, resulting in the formation of cavities (pores).

For removing the porogenic leachable material from the mixture, it is expedient for said material to be soluble in at least one solvent and to be essentially insoluble in at least one further solvent. A material is essentially insoluble when, in particular, it is less than 30% by weight, preferably less than 20% by weight, in particular less than 10% by weight, for example less than 5, 4, 3, 2 and 1% by weight, soluble under the processing conditions, i.e. as a rule at temperatures in the range from 18 °C to 25 °C and under atmospheric pressure.

The structure and properties of the resulting primary structures are essentially determined by the size, shape and weight ratio of porogenic particles which are used for preparing them. Since the size of the porogenic particles will affect the size of the pores formed upon leaching of the porogenic leachable material, it has generally an average grain size of from 100 to 400 $\mu$m, preferably from 300 to 400 $\mu$m. This size will correspond approximately to the size of the pores formed by the leaching of the porogenic particles.

In this connection, it is to be noted that not only the nature of the porogenic leachable material which is important but also, especially, the grain size distribution of the porogenic particles. Thus, it applies, in general, that not only the pore size but also the connectivity, i.e. the network of cavities which communicate with each other, increase as the grain size increases. This network, which is also termed macrostructure or macroporous structure, is to be distinguished from the pores which can be obtained by foaming and which are as a rule closed and therefore form a structure which is designated a microstructure or microporous structure.

For the inventive preparation process, the porogenic leachable material is preferably a salt, more preferably selected from the group consisting of sodium chloride, sodium citrate, sodium tartrate, and potassium chloride, most preferably sodium chloride. Sodium chloride is particularly beneficial since it is widely available to a low prize, poses no harm to the human body and is therefore easy to handle. In addition, sodium chloride is soluble in a variety of solvents, including water.

For the preparation of the matrix, the primary structure is then immersed in a solution (PS-II) comprising the second polymer component dissolved in a solvent (S-II) (step d).

According to the present invention, the second polymer component is selected from the group consisting of collagens, laminin, fibronectin and mixtures thereof. From these, collagen is preferred.

Thus, any type of solvent that allows for solubilizing the second polymer component is suitable for use in the method of the present invention. Acidic aqueous solutions have been found particularly expedient for this purpose.

In a preferred embodiment, in step d), the primary structure is immersed in an acidic aqueous solution comprising the second polymer component, preferably under reduced pressure. The immersion under reduced pressure enables the primary pores of the primary structure to be fully filled with the solution comprising the second polymer component.

In a particularly preferred embodiment, the primary structure is immersed in type I collagen acidic solutions (pH 3.2), preferably under vacuum so that the pores of the primary structure are filled with collagen solution. The effective concentration of collagen in the solution is preferably in the range from 0.1 to 1.5 (w/v)%.Upon removal of the solvent (S-II) in step e), the matrix according to the present invention is obtained. Organic solvents are preferably removed under reduced pressure. Preferably, a centrifugation step is performed before the removing the solvent (S-II) under reduced pressure.

Aqueous solvents are preferably removed by freezing at -80 °C and lyophilisation under vacuum. Times of about 3 h to 60 h, and, in particular, in the range of from about 12 h to 36 h, under vacuum have proved to be expedient in this connection.

In a particularly preferred embodiment, the solvent (S-II) is an aqueous solvent and removal of the solvent (S-II) in step e) of the inventive method involves the subsequent steps of

e1) centrifugation; and

e2) freeze-drying and/or lyophilisation under vacuum.

The centrifugation step serves to remove any excess of the second polymer component containing solution (PS-II) that is not adhering to the surface of the primary structure. By freeze-drying and/or lyophilisation under vacuum, the solvent (S-II) is essentially completely removed and in the resulting matrix structure, the second polymer component forms a three-dimensional secondary structure which is contained within the interior space of at least a part of the primary pores.

The amount of secondary structure contained within the primary pores can be regulated by adjusting the viscosity of the solution (PS-II) comprising the dissolved secondary polymer compound and/or the centrifugation rate. For instance, the higher the viscosity of the solution (PS-II) and/or the centrifugation rate, the less secondary structure will be formed within the primary pores. Generally, for avoiding the formation of excess secondary structure within the primary pores, the centrifugal rate (or centrifugal acceleration) is preferably set above 500 g. More preferably, the centrifugal rate is set within the range of 600 g and 10'000 g.In addition, it is preferred that the concentration of the second polymer component dissolved in the solution (PS-II) is in the range from 0.1 to 1.5 (w/v)%. Within this range, the viscosity of the solution (PS-II) allows for essentially complete infiltration of the solution (PS-II) into the primary pores.

In order to obtain a highly cross-linked three-dimensional secondary polymer structure, the matrix can further be treated with a cross-linking agent such as glutaraldehyde.

It is further preferred that the matrix is subjected to an additional step f) involving a surface modification step, in particular a plasma treatment step.

As mentioned, the present invention relates to a matrix according to the invention for therapeutic use, and in particular, for use in tissue engineering in general, and more specifically, for use in the preparation of an implant, in particular a liver implant.

In the field of tissue engineering, the matrices according to the invention may serve as scaffolds into which cells migrate and/or onto which cells adhere. In tissue engineering, this combination of matrix and cells adhering to the latter is generally referred to as "implant".

Matrices according to the invention have proven to exhibit crucial advantages when used in an implant for tissue engineering. For instance, the internal dimensions, meaning the internal free space, enable the matrices to be efficiently colonized with cells. The matrices are, on the one hand, freely moldable and, on the other hand, provide adequate stability and rigidity for withstanding the surgical implantation procedure and resisting the mechanical forces acting at the implantation site. The initial cell destruction, which sets in after the implantation, is limited, and, after a short time, implanted tissue can assume the intended function. Shortly after the implantation, blood vessels or blood vessel-rich granulation tissue, and also nervous tissue, begin to proliferate into the implant. Furthermore, the matrices according to the invention can be prepared without having to use physiologically harmful solvents, for example formaldehyde, which means that no special method is required for eliminating the solvents and there is no danger of residual quantities of these solvents remaining.

For this, the matrices can, for example, be inoculated with the desired cells *in vitro,* i.e. treated with a cell-containing solution and incubated until cells have adhered to the matrix. Such a matrix together with cells adhering to it (referred to here as an implant) can then be subjected to further procedural steps, for example further culturing, where appropriate under the influence of active compounds, e.g. for the purpose of further expanding the cells or modifying their properties, and/or be stored until implantation in a suitable manner, for example on ice or in a bio-flow reactor under standard conditions. In the context of this use, it is advantageous to be able to initially isolate, and, where appropriate, also expand, in vitro the cells which are intended for the implantation. In particular, this thereby makes it possible to apply different cell types, such as the above-described hepatocytes together with cells of Islet of Langerhans, to a matrix.

As indicated above, the matrix of the present invention has shown to be not only well suited for cell cultivation in general but in particular for the cultivation of hepatocytes, which are known to be highly difficult to cultivate: Although the liver has an amazing capacity for regeneration in vivo, hepatocytes in culture have limited proliferation capacity and do normally not survive for more than a few hours in suspension. The problem is that freshly isolated hepatocytes exhibit the typical structure and most of the functions of their in vivo counter-parts but they have lost specialized membrane domains, e.g. intercellular junctions and bile canaliculi, such that they cannot survive without attaching to a substrate. Yet, even when these hepatocytes are plated in conventional culture conditions such that they could reaggregate and reconstitute bile canaliculus-like structures, they exhibit early phenotypic alterations and survive for only a few days.

Even more difficulties arise when hepatocytes are to be loaded onto a porous substrate, e.g. for use as implant: Due to their small size (10 to 15 $\mu$m compared to e.g. fibroblasts of about 50 $\mu$m), the loading of hepatocytes bears the risk that cells fall through the porous substrate instead of being retained within. This problem is aggravated by the fact that the materials which are generally used for the fabrication of such matrices, such as PLA, PLG, PLGA, etc. are highly hydrophobic and thereby hamper any cell attachment.

It has surprisingly been found that the matrix of the present invention is particularly well suited for hepatocytes cell cultivation. Without wanting to be bound by the theory, it is assumed that this is at least partially owed to the fact that the inventive matrix comprises primary pores which are at least partly filled with the secondary porous structure. As such, the matrix allows for also retaining cells of small size, such as hepatocytes. By increasing or decreasing the content and/or the porosity of the second polymer component, the space available inside the primary pores can be adjusted to the needs for cell proliferation. In this regard it is particularly preferred that the second polymer component forms a secondary structure with a porosity of at least 90%.

The content of the second polymer component can be adjusted, for instance, by adjusting the concentration of the second polymer component dissolved in the solution (PS-II) in which the primary structure is immersed during the preparation of the matrix described above. Most preferably, the concentration of second polymer component in the solution (PS-II) is in the range from 0.1 to 1.5 (w/v)%.

In addition, the secondary polymer component being selected from the group of collagens, laminin, fibronectin and mixtures thereof provides the matrix with excellent hydrophilic properties (i.e. improved wettability with water). The increased hydrophilic properties of the inventive matrix greatly facilitate cell seeding in the porous matrix structure since cells can more easily infiltrate into the matrix and adhere to the secondary structure, thereby preventing leakage of the seeded cells.

From the above-mentioned materials that are used as secondary polymer component, collagen, in particular gelatine, is most preferred. Owing to its nano-fibrous architecture, it is particularly effective in promoting cell adhesion, growth and differentiated function in tissue cultures.

Apart from the special material composition given by the first and secondary polymer component, it is assumed that the specific three-dimensional structure of the inventive matrix provides an ideal structural environment for adhesion and cultivation of cells within the matrix. For instance, the size of the pores and the primary pores in particular, is preferably such that the matrix can not only be loaded with single cells but with multicellular cell clusters, also called "spheroids".

The possibility to load the matrix with multicellular cell clusters is a particular advantage when it comes to hepatocytes since it has been found their mortality rate can be significantly decreased if the hepatocytes are loaded onto a substrate in a preassembled state. Therefore, taking into account the mean cell size of hepatocytes (10 to 15 $\mu$m) and in view of the matrix' use for preparing a liver implant, the primary pores have preferably an average pore size diameter of 150 $\mu$m to 300 $\mu$m, preferably 200 $\mu$m to 300 $\mu$m.

A few years ago, a new cultivation technique has been developed which allows for providing such pre-assembled cell clusters in a transferable and also conservable state. This cultivation technology has become known under the name of the "hanging drop technology", which involves the cultivation of cells in drops of culture media that are hanging at a surface. These drops of culture media are inoculated with living cells and, under the pull of gravity, the cells in the culture media droplets descend to assemble as a miniature tissue, which results in multicellular micro-tissue spheroids that are morphologically and functionally very similar to native tissue. These pre-prepared micro-tissues can either be purchased ready-to-use or can be produced, for instance, in accordance with the GravityPLUS™ technology developed by InSphero, a spin-off company of the Swiss Federal Institute of Technology (ETH) Zurich and the University Zurich, Switzerland. It is hereby referred to the disclosures of the European Patent No. EP-B1-2342317, relating to InSphero's hanging-drop technology.

In particular in view of using the above multicellular micro-tissue spheroids, the inventive matrix is particularly well suited as the pores of the matrix, preferably having primary pores with an average pore size diameter of 150 $\mu$m to 300 $\mu$m, provide sufficient space that the preassembled cell clusters readily fit into the primary pores.

Although the implantation of an implant that has been loaded with living cells beforehand (*in vitro* inoculation) is often preferred, another possibility is to implant the matrix (without any prior cell adhesion) with the aim of inducing precursor cells, which are capable of tissue regeneration, to migrate into a damaged tissue and there regenerate tissue which has been lost. For this, the matrix must be configured such that desired cells, but not undesired cells, can migrate into the matrix. Such a use is generally described as being guided tissue regeneration (GTR).

A matrix according to the invention or an implant according to the invention can therefore be used for treating the human or animal body. For this, one or more matrices, or one or more implants, is/are introduced into the body to be treated by way of a surgical or interventional procedure. If the implant contains cells having an organ function, or if cells having an organ function are to migrate into the matrix, as is the case, for example, with hepatocytes or Langerhans islet cells, the matrices or implants can, for example, be implanted into the mesenterium, subcutaneous tissue, retroperitoneum, properitoneal space or intramuscular space of the individual to be treated.

In principle, any individuals who require an appropriate tissue replacement can be treated with the matrices or implants according to the invention. These individuals are as a rule individuals who are suffering from a specific disturbance or disease whose course involves the loss of functional tissue. This may potentially affect whole organs, for example the liver or the pancreas.

It is therefore another object of the present invention to provide an implant comprising a matrix and cells embedded therein for use in tissue engineering.

This object is achieved by the subject-matter according to claim 1, pertaining to an implant for tissue engineering which comprises at least one matrix of the present invention and at least one cell of Islet of Langerhans. Cells of Islets of Langerhans are cells from the pancreas where they form cell aggregations - so-called "islets" of Langerhans cells. There are about one million islets distributed throughout the pancreas of a healthy adult human, each of which measures from less than 40 $\mu$m to 400 $\mu$m in diameter and contains a number of cells ranging from only a few cells to about 5000 cells per islet (Bonner-Weir 1994). 65 to 80% of the cells in human Islets of Langerhans are insulin-producing beta cells, which are distributed throughout the islet.

The structure of the matrix renders it an excellent substrate for cells and it is possible to culture a single cell type or several different cell types on the matrix. In this connection, apart from cells of Islet of Langerhans, the implant may comprise other cells, in particular, from liver cells, pancreas cells, fat cells, intestine cells, skin cells, blood vessel cells, nerve cells, muscle cells, smooth muscle cells, thyroid gland cells, urothelian cells, cells from the fallopian tubes, urogenital tissues, fibroblasts or fibrocytes and tooth root cells.

The present pores in the matrix provide space for cellular ingrowth and the deposition of extracellular matrix. Since the matrix provides the necessary mechanical stability and elasticity, cell culture, after a static phase at the beginning, may be done dynamically. This means, cells are for instance cultured in a static environment in cell culture flasks or the like. Subsequently, the cells growing on the scaffold are transferred in perfusion chamber that imitates a dynamic environment. This process influences the further cell growth. For instance, the composition of the extracellular matrix depends on the mechanical strain the cells are exposed to.

It is particularly preferred that the implant further comprises hepatocytes in addition to the cells of Islet of Langerhans. In this embodiment, the implant therefore comprises at least two cell types, which are preferably autologous cells (cells from the individual receiving the later implant) but may alternatively also be heterologous.

It has surprisingly been found that implants according to the present invention which comprise hepatocytes and cells of Islet of Langerhans provide excellent biological substitutes of liver tissue that allows for restoring, maintaining and improving the organ function of the liver in case of chronic or acute liver disease. In particular, specific ratios of hepatocytes to cell of Islet of Langerhans have been found to be particularly advantageous for allowing the implant to fulfil its particular function.

In a particularly preferred embodiment, the implants have been loaded with multicellular micro-tissue spheroids comprising pre-assembled cell clusters of hepatocytes and at least one cell, preferably multiple cells, of Islet of Langerhans. Such pre-assembled cell clusters, more particularly pre-assembled cell clusters in the form of multicellular micro-tissue spheroids, are preferably prepared in accordance with the hanging-drop technology disclosed in EP-B1-2342317, for instance. Alternatively, readily prepared hanging drops containing the pre-assembled cell clusters can be purchased as pre-prepared spheroids which are conserved in a frozen state, preferably at -78 °C.

The preassembled cell clusters or micro-tissues spheroids have preferably a size of 100 $\mu$m to 300 $\mu$m in width and height. As such, the preassembled cell clusters readily fit into the primary pores when the matrix is loaded with the cells.

The invention therefore most preferably also relates to implants which, after implantation, carry out metabolic functions of a liver. For this purpose, the ratio of hepatocytes to cells of Islets of Langerhans is preferably $1 \times 10^6$ hepatocytes to 20'000-500'000 cells of Islets of Langerhans, more preferably $1 \times 10^6$ hepatocytes to 20'000-100'000 cells of Islets of Langerhans. Most preferably, the implant comprises $1 \times 10^6$ hepatocytes to 20'000-70'000 cells of Islets of Langerhans.

In this regard it is to be noted that the above numbers explicitly relate to the number of cells and not to the number of preassembled cell clusters.

In addition, the invention preferably relates to implants which, after implantation, exhibit the endocrinal properties of an equivalent pancreas organ. For performing the function of the pancreas, the implant comprises preferably more cells of Islets of Langerhans than hepatocytes. In this regard, a preferred ratio of hepatocytes to cells of Islets of Langerhans lies in the range of 1:1 to 1:200. Most preferably, for this purpose, the ratio of hepatocytes to cells of Islets of Langerhans is about 1:100.

The cells or cell mixtures which are to be used for colonizing matrices according to the invention can be obtained in a manner known per se. For the purpose of producing an autologous implant, the cells are preferably derived from the individual into whom the implant is to be inserted. Thus, suitable tissue, for example a portion of liver or pancreas, is as a rule removed from the individual and prepared in a suitable way for the inoculation and culturing the matrix in vitro. In this connection, it is of importance that the cells exhibit a vitality rate which is as high as possible.

If liver cells are being obtained from the liver tissue, account has to be taken of the fact that the liver cells are surrounded by a thick connective tissue layer, particularly in the case of a liver cirrhosis. According to the invention, solutions of defined composition are used in order to be able to isolate the liver cells containing a proportion of vital cells which is as high as possible.

Preferably, an aqueous composition A which contains NaCl, KCl, $Na_3PO_4 \cdot 12\ H_2O$, glucose and ethylene glycol tetraacetic acid (EGTA), and has a pH of about 7.4, is used for perfusing a portion of liver or pancreas. In particular, 1000 ml of this solution contain about 8 g of NaCl, 0.2 g of KCl, 0.27 g of $Na_3PO_4 \cdot 12\ H_2O$, 1.8 g of glucose and 1.9 g of EGTA. The perfusion is preferably carried out at a temperature of about 37 °C and a flow rate of about 15 ml/min. A few minutes, in particular from about 3 to 5 minutes, for example about 4 minutes, are sufficient for perfusing the tissue portion adequately at the abovementioned flow rate.

Alternatively, it is also possible to use an aqueous composition A' which contains EGTA for perfusing a portion of liver or pancreas.

For perfusing a portion of liver or pancreas, use is preferably made of an aqueous composition B which has a pH of from about 7.3 to 7.4, preferably about 7.35. Composition B is preferably low glucose DMEM (Dulbecco's modified Eagle's medium) containing HEPES, $CaCl_2$ and collagenase type IV 50 U/ml.

In this case, too, perfusing at about 37° C. and a flow rate of about 10 ml/min has proved to be expedient. A few minutes, in particular from about 5 to 10 minutes, for example about 6 to 7 minutes, are sufficient for perfusing the tissue portion adequately.

Alternatively, it is also possible to use an aqueous composition B', which contains collagenase and hyaluronidase, for perfusing a portion of liver or pancreas. 1000 ml of the solution preferably contain 50 U of hyaluronidase/ml.

It is advantageous for the vitality of the cells to be isolated if the tissue portion is initially treated with composition A and then treated with composition B. Alternatively, it is possible to use a composition A' initially and then to use a composition B'.

Following on from the perfusion, the tissue portion can then be chopped and carefully suspended in a suitable medium, for example Williams medium E. If the resulting cell suspension still contains relatively coarse cell debris, the latter can be removed in a manner known per se, for example by filtering the cell suspension through a nylon net (100 $\mu$m). The cells of the filtrate can then be pelleted carefully, in connection with which a three-minute centrifugation at 60-130 g and 4-22 °C has proven to be advantageous.

The cells which have been isolated are loaded onto the matrices in a manner known per se. As a rule, the cells are loaded onto the matrix as a cell-containing solution and the cells and matrix are then incubated, usually under cell culture conditions, until cells adhere to the matrix. If cells of Islets of Langerhans are provided on the matrix with at least one other cell type, for example hepatocytes, the different cell types can in principle be loaded jointly or else consecutively onto the matrix.

The present invention therefore also provides a method for preparing an implant, comprising the steps of

a. providing a porous matrix according to the present invention, and

b. loading hepatocytes and at least one cell of Islets of Langerhans onto at least one side the matrix.

More specifically, cells of Islets of Langerhans are preferably loaded on initially, followed by hepatocytes, with an incubation in each case being carried out after the loading until at least a part of the cells is adhering to the matrix.

Most preferably, cells of Islets of Langerhans and hepatocytes are loaded onto the matrix jointly, in preassembled cell clusters. Such pre-assembled cell clusters can be purchased or cultured, for instance, by using the hanging-drop-technology described above.

In a particularly preferred embodiment, the preassembled cell clusters or micro-tissues are provided with a size of 100 $\mu$m to 300 $\mu$m in width and height, such that they readily fit into the primary pores when the matrix is loaded with the cells.

The matrix of the present invention does, however, not only facilitate the deposition of the cells thereon, but also provides a structural environment that allows not only for the cells to survive but also to proliferate and migrate within the matrix. More specifically, the inventive matrix allows for the transport of liquids and (macro-)molecules into the matrix or even pass through the matrix. In the beginning, exchange of nutrients (e.g. oxygen) and waste takes place by means of diffusion. However, diffusion as a passive transportation means for living processes is effective only over short distances. Specifically, diffusion has been found to enable oxygen and nutrients supply to cells within the matrix, but only to maximum distance of about 400 $\mu$m from the surface to the inside of the matrix. This means that cells which have been deposited on the matrix can usually not migrate deeper than this diffusion distance into the matrix due to lack of essential nutrients, especially oxygen. Taking account of the limited diffusion range, the matrix has preferably a thickness of 0.1 mm to 1.0 mm, more preferably 0.5 mm to 1.0 mm.

The implant of the present invention, i.e. the matrix with cells deposited thereon, on the other hand, is not necessarily limited to the dimensions of the matrix. If an implant with a thickness of more than 1.0 mm is desired, e.g. for stability reasons or for providing a larger attachment site for the cells, the implant can be prepared from at least two matrices that are laid on top of each other and optionally glued or sutured together. Such a "bi- or multi-layered implant" is preferably prepared from at least two matrices which have been loaded with cells prior to the assembly.

In accordance with a particularly preferred embodiment, the implant of the present invention is prepared as a bi- or multi-layered structure that forms an integrally stable body by the following procedure:

a) a matrix according to the present invention is provided, the matrix being essentially in the form of a sheet with a thickness in the range of about 0.1 to 1.0 mm;

b) cells - in particular hepatocytes and at least one cell of Islets of Langerhans - are loaded onto at least one side the matrix;

c) the matrix with the cells thereon is folded to a multi-layered structure.

Before the matrix is folded in step c), the matrix is preferably placed in an incubator which provides optimal conditions for cell proliferation. The preferred thickness of the matrix defined above ensures that the cells can sufficiently be provided with oxygen and nutrients by diffusion such that they can proliferate and form functional tissue, such as transport channels, to enable also active transport of substances throughout the matrix structure. When the matrix is then folded to a multi-layered structure (step c)) transportation of substances can also take place actively, through the newly formed functional tissue throughout the matrix. In addition, since the matrix is preferably folded rather than assembled from several individual matrices, the resulting implant is particularly stable and allows for facilitated transportation and implantation without risk of disassembly.

Most preferably, in step b), cells are loaded onto both sides of the matrix, i.e. on the upper and the under side. This allows migration into the matrix from the top and from the bottom. The loading of the matrix with cells on both sides can be achieved, by loading the first side of the matrix with cells and then turning the matrix upside down for loading the other side of the matrix with cells. This procedure can be repeated several times, incubating the matrix for a short time in between in order to allow the cells to migrate into the matrix before further cells are loaded onto the surface of the matrix.

It is particularly preferred that a multi-layered implant is provided with a total thickness in the range of 1 mm to 10 mm. For instance, a matrix of 0.6 mm in thickness is preferably folded twice times to obtain a three-layered implant with a total thickness (or height) of 1.8 mm.

It is further possible to produce a multi-layered implant from more than one matrix. For instance, a smaller matrix may be placed in the center of a second, larger matrix and the edges of the second matrix are subsequently folded around the inner smaller matrix. In other words, a smaller first matrix can be enclosed by a second matrix that is "wrapped around" the first matrix. This way, the first matrix can be inoculated with a first cell type and the second matrix with a second cell type before the second matrix is folded around the first matrix.

The present invention is directed, in particular, towards treating diseases which lead to chronic liver or pancreatic disease and/or chronic liver and pancreatic failure. These diseases include, for example, chronic hepatitis and biliary cirrhosis in adults as well as biliary atresia and congenital metabolic defects in children. In fact, the invented matrix can be used to relieve a disease in all cases where a liver transplantation is indicated and in all cases of liver impairment or under-function. On the other hand, a pancreas transplantation is indicated, in particular, in the case of all forms of diabetes mellitus, in particular type I or type II diabetes mellitus. For treating pancreatic disease or pancreatic failure, the implant is preferably provided with hepatocytes and cells of Islets of Langerhans, most preferably in a ratio of about 1 hepatocyte to about 100 cells of Islets of Langerhans.

The present invention therefore also relates to the use of a matrix according to the invention or of an implant according to the invention in making available a therapeutic agent for carrying out a transplantation on an individual and, in this connection, in particular for treating an individual who is suffering from an at least partial loss of functional tissue which is to be replaced by the transplant.

In view of the matrix, preferred use in the field of liver transplantology, the present invention further provides a kit for the preparation of a liver implant. Said kit comprises

i) at least one matrix of the present invention and

ii) multicellular micro-tissue spheroids comprising pre-assembled cell clusters in culture media.

According to a particularly preferred embodiment, the kit comprises at least one matrix of the present invention and pre-assembled cell clusters of at least one cell of Islets of Langerhans and hepatocytes in culture media.

The multicellular micro-tissue spheroids are preferably prepared in accordance with the above-described "hanging-drop-technology", for instance in accordance with the GravityPLUS™ hanging-drop technology patented by InSphero AG Switzerland.

Without limiting the present invention to the following applications, matrices according to the present invention can be used, for instance, for the transplantation of fibroblasts, muscle cells or mucosa cells. Transplantation of mucosa cells is of particular interest in surgical procedures involving the small bowel.

## Materials and Methods

### Matrix Preparation

Matrices of 20 mm in diameter and of 2 mm thickness were prepared by the following procedure:
Example IThe three-dimensional primary structure was prepared from PGLA by a particulate-leaching technique using sieved sodium chloride particulates.

Poly(DL-lactic-co-glycolic acid) (PLGA 75:25), with a molecular weight of 90-126 kD, was purchased from Evonik Industries AG (Essen, Germany). PGLA pellets were frozen in liquid nitrogen ($N_2$) and shredded at 10'000 rpm for 2 minutes (Däschler impact system). The shredding process was carried out in a ball mill and sieved after milling. The particles thus obtained had an average diameter of 108-250 $\mu$m.

2 g of the polymer pellets were dissolved in 50 ml chloroform at a temperature of 22.5 °C for 80 minutes, yielding polymer solution (A).

In a next step, pure NaCl was shredded at 10'000 rpm for 2 minutes. The particles thus obtained had an average diameter of 108-390 $\mu$m.

0.5 g of the shredded polymer pellets and 48 g of the shredded NaCl particles were mixed at 90 rpm for 4 minutes, stowed with the polymer solution (A) and placed in circular ceramic platelets. The solvent was vaporized at 35 °C for 28 hours. The solvent vaporization was carried out in a controlled temperature oven, placed in a fume hood to extract chloroform toxic gases. The remaining salt/polymer composite was pressurized in a high pressure molding press at 1000 psi for 1 min before the NaCl was leached out by washing the composite with deionized water until the weight of the dried primary structure remained unchanged. The thus formed product of a three-dimensional primary structure with primary pores was dried at 40 °C for 12 hours. SEM analysis demonstrated that the primary structure had an uniformly distributed and interconnected pore structure. The pore size and morphology were the same as those of the NaCl particulates used.The primary structure was then immersed in type I collagen acidic solution (1.0 (w/v)%, pH 3.2, purchased from ICN Biomedicals, Costa Mesa, California) under a vacuum so that the primary pores filled with collagen solution. The collagen solution-containing primary structure was subsequently frozen at -80 °C for 12 hours and lyophilized under a vacuum of 0.2 torr for an additional 24 hours to allow the formation of a secondary collagen structure in the primary pores. The collagen structure was further cross linked by treatment with glutaraldehyde vapour saturated with 25 % glutaraldehyde aqueous solution at 37 °C for 4 hours. After being cross-linked, the matrix was treated with 0.1 M of glycine aqueous solution to block non-reacted aldehyde groups. After being washed with deionized water and lyophilized, the matrix comprising the primary structure with primary pores and a three-dimensional secondary collagen structure with secondary pores contained within the primary pores was obtained as the final product.

*Example 2*

Sieved NaCl particulates (36.0 g) ranging in diameter from 355 to 425 $\mu$m were put in an aluminum pan and stowed with a PLGA solution in chloroform (17.8 ml) at a concentration of 27 (w/v)%. Thus, PLGA was dissolved in chloroform and then cast onto an aluminium pan filled with the NaCl particulates as porogen.

The mixture was air-dried in an air draft for 48 h followed by 48 h of vacuum drying. The dried PLGA/NaCl blocks were then detached from the aluminum pan and soaked in deionized water to leach out the NaCl particulates. The washing was continued for 20 times, with 500 mL deionized water for 1 h each, to completely remove the NaCl particulates. Finally, PLGA primary structures were formed after air-drying. The porosity of the PLGA sponges was determined by a mercury intrusion porosimeter (AutoPore IV, Shimadzu, Kyoto, Japan). The PLGA primary structures were cut into small pieces of 6.0 mm × 6.0 mm × 5.0 mm for surface treatment.

An aqueous 0.5 (w/v)% collagen solution (porcine type I collagen, isolated by enzyme treatment) was prepared by diluting an aqueous 1.0 (w/v)% collagen solution with an aqueous solution of hydrochloric acid (0.001 M) to a collagen concentration of 0.5 (w/v)%. The pH of the aqueous 0.5 (w/v)% collagen solution was set to 2.5-3.5.

The cut PLGA primary structures were immersed in the aqueous 0.5 (w/v)% collagen solution under reduced pressure so that the primary pores of the primary structures were filled with collagen solution. The PLGA primary structures containing the collagen solution were placed on cell strainer membranes that were placed in 50 mL centrifugation tubes and centrifuged for 10 min to remove any excess collagen solution at various centrifugal accelerations of 96, 600, 1200, 2400, 5000, and 10,000g using a multipurpose refrigerated centrifuge LX-120 (Tomy Seiko Co., Tokyo, Japan) or a high-speed refrigerated centrifuge (Himac CR 21G, Hitachi, Tokyo, Japan) operated at 4 °C. After centrifugation, the collagen-treated PLGA structures were frozen in a freezer at -80 °C for 4 h. The frozen porous scaffolds were freeze-dried under a vacuum of less than 25 Pa for 24 h. After freeze-drying, the collagen-coated PLGA structures were stored in a desiccator until use.

**Matrix properties analysis**

*Porosity calculation*

Porosity of the matrix was determined by the following formula:

$$\text{\O} = 1 - (\text{Solid Volume})/(\text{Total Volume})$$

The porosity of three batch production process matrices is given in table 1

Table 1

| batch | porosity [%] |
|-------|--------------|
| $\text{\O}_1$ | 97.4 |
| $\text{\O}_2$ | 99.5 |
| $\text{\O}_3$ | 99.2 |

*Collagen content analysis*

The PLGA primary structures were weighed before immersion in the collagen aqueous solution and the wet weights of the collagen-treated PLGA structures were weighed after centrifugation using an analytical balance (AG 135, Mettler-Toledo, OH). The differences between the two weights indicated the amount of collagen aqueous solution that remained in the PLGA sponges after centrifugation.

*Hydrophilicity analysis*

The hydrophilicity has been tested as follows: 300 $\mu$l Phosphate Buffered Saline (PBS; Sigma) was pipetted on top of the matrix in a drop-wise manner in order to determine whether the liquid is absorbed or repelled by matrix.

*Cell adherence tests*

The cell adherence on the matrix was tested by coating the matrix with collagen type 1 and pipetting 300 $\mu$l of cell suspension on top of the matrix in a drop-wise manner. Then, the matrix loaded with cell suspension was incubated at 37 °C, 5% $CO_2$ and 95% relative humidity. The cells that remained in the suspension and therefore not attached to the matrix were counted after one and three days while changing the media.

*Cell number calculation*

The cell number was determined with a haemocytometer (Neubauer Improved Chamber) after having stained the cell suspension with 0.2% Tryptan blue solution for 2 min.

The number of attached cells was determined according to the following formula:

$$[number\ of\ cells\ seeded\ onto\ the\ matrix] -$$

$$[number\ of\ cells\ remaining\ in\ the\ wells]$$

$$= number\ of\ cells\ adherent\ onto\ the\ matrix.$$

**Cell Isolation**

Abbreviations and substances:

- EGTA: ethylene glycol tetraacetic acid (Sigma E4378)
- Collagenase type I: (Worthington 4196; 246 U/mg)
- FCS: fetal calf serum (Sigma F2442)
- HEPES: 4-(2-hydroxyethyl)-1-piperazineethanesulfonic-acid (Sigma)
- Williams Medium E: standard medium for cultivating hepatocytes comprising "William E Medium (Sigma W4128) and 10 vol.-% of serum of the donor or 10 vol.-% FCS

A portion of liver was removed, in a manner known per se, from the individual to be transplanted. The liver portion which has been removed was first of all perfused for 7 minutes, at a flow rate of 30 ml/min and at 37 °C., with a solution (8 g of NaCl; 0.2 g of KCl; 0.266 g $Na_3PO_4 \cdot 12\ H_2O$, 1.8 g of glucose and 0.19 g of EGTA ; made up to 1000 ml with distilled water; pH 7.4). The liver portion was then perfused for a further 10 min, at a flow rate of 30 ml/min and at 37 °C., with a collagenase/hyalorunidase solution (8 g of NaCl; 0.2 g of KC1; 0.266 g $Na_3PO_4 \cdot 1.2\ H_2O$, 1.8 g of glucose, 0.735 g $CaCl_2$ and 5-10 ml of HEPES; 10 U/ml of collagenase; made up to 1000 ml with distilled water; pH 7.2). After the perfusion had come to an end, the liver portion was chopped and suspended carefully in Williams medium E. The cell suspension was filtered (nylon net; 100 $\mu$m) and then washed with Williams medium E. After that, the cells were centrifuged at 100 g at room temperature for 5-10 min. The viability of the cells, which was determined using Tryptan Blue (0.2%), was 70%.

The viability of the cell suspension was determined using the trypan blue dye exclusion test and by using the following formula:

$$Viability\ in\ \% = \frac{counted\ viable\ cells\ x\ 100}{total\ cell\ number}$$

Cells of Islet of Langerhans were isolated from a portion of pancreas in the same way.

**Cell Colonization**

In the first step, the matrices were incubated with hepatocytes together with cells of Islet of Langerhans which were isolated as described under "Cell isolation" above.

For this, a hepatocyte solution and a Islets of Langerhans cell solution were prepared by suspending hepatocytes and cells of Islets of Langerhans, respectively, in warm Williams E medium, completed with serum and the cell concentration of each solution was adjusted to about 1 to $3 \times 10^6$ cells/ml. The cell number was determined by counting it in a 0.25 mm counting tube under an inverted Olympus microscope. 18 ml of hepatocyte solution and 2 ml of pancreatic Islets of Langerhans cell solution were united carefully resuspended in order to obtain 20 ml cell solution for the autologous transplantation. The ratio of hepatocytes to cells of Islets of Langerhans in the mixed cell solution was thus in the range of 9:1.

20 matrices were prepared in a multiwall plate and 1 ml of the above prepared mixed cell solution (comprising a total number of $1 \times 10^6$ to $3 \times 10^6$ cells) were then applied to each matrix using a pipette. The matrices which had been treated in this way were then placed, for 1 hour in a cell culture incubator in order to allow the cells to adhere. Then, another 1 ml of warm Williams E medium, completed with human serum was pipetted carefully in each well of the multiwall plate as an additional supply for the cells. The matrices were incubated at 37 °C, at 5% $CO_2$ and 95% rel. humidity for 24 to 36 hours.

The cell number per matrix was determined after incubation to determine the adherence rate of the implanted cells on the matrix. Adherence rates were in the range of 30% to 66%

Prior to implantation, the matrices could be kept for about 1.5 hours on ice. If an implantation was planned for a later time, the matrix could be stored under standard conditions in a bioflow reactor for up to 5 days.

**Secretory Activity and Rate of Proliferation of the Hepatocytes**

Lewis rats were transplanted with cell-colonized matrices prepared in line with example 3 in an orthotopic or heterotopic implantation procedure. The transplants were removed once again from the animals at various times and examined morphometrically. The number of cells in the transplants, which exhibited the form of a circular disc having a diameter of 15 mm and a thickness of 2 mm, was $94 \times 10^3$, $140 \times 10^3$ and, respectively, $146 \times 10^3$ at 1, 6 and 12 months after transplantation.

Histo-chemical analysis proved albumin secretion from cells on the implant. Secretion has been found to start after two weeks. Hepatocytes from the transplant which was removed one month after transplantation exhibited normal expression of albumin. Proliferating hepatocytes were found in all the preparations without there being any pathological increase in proliferation rate. The hepatocytes which had been transplanted in accordance with the invention exhibited an incorporation of BrdU which was increased by a factor of 3 as compared with that of standard liver preparations. After implantation, the metabolic activities of the livers were investigated.

**Vascularization**

Further investigation of the matrices prepared in line with example 3 showed that these matrices were outstandingly well vascularized only one month after implantation. The blood vessels extended macroscopically to the matrix and the transplanted hepatocytes and cells of Islet of Langerhans attained contact as a result of adequate capillarization, with the cardiovascular system of the transplant recipient.

It could furthermore be observed that the cotransplanted cells of Islet of Langerhans did not cause any hypoglycemia in the recipient. The endocrine secretion performance of these cells, and also of the recipient's own islet cells, was presumably regulated by a feedback mechanism.

**Adoption of Liver Function**

Gunn rats are regarded as being an animal model for the human Crigler-Najar syndrome since, as a result of a specific congenital metabolic enzyme defect, their livers are unable to conjugate bilirubin adequately. As a consequence, toxic blood plasma levels of unconjugated bilirubin lead to death as the result of substantial consequential damage.

Three Gunn rats were transplanted with a cell-colonized matrix prepared in line with example 3. The matrix had an external area of in all 10 cm².

The bilirubin level in the experimental animals fell only four weeks after transplantation. Bilirubin was from now on being conjugated. In all three cases, the conjugated bilirubin could be detected, using a bile duct probe, in the bile ducts of the liver which was still present. Consequently, the bilirubin which was conjugated in the matrix reached the liver hematogenically and could be eliminated from the liver by way of the bile duct system.

**Claims**

1. An implant for tissue engineering, comprising

    - at least one porous matrix for tissue engineering, the matrix comprising a first biodegradable and biocompatible polymer component forming a three-dimensional primary structure with primary pores, and further comprising a second biodegradable and biocompatible polymer component other than the first polymer component selected from the group consisting of collagens, laminin, fibronectin and mixtures thereof, wherein the second polymer component forms a three-dimensional secondary structure with secondary pores, the secondary structure being contained within the interior space of at least a part of the primary pores; and

- at least one cell of Islets of Langerhans,
wherein the matrix is essentially in the form of a sheet with a thickness in the range of 0.1 to 1.0 mm and is provided with cells on at least one side, and wherein the implant has a bi- or multi-layered structure provided by folding the matrix or by laying two or more matrices on top of each other.

2. Implant according to claim 1, wherein the first polymer component of the matrix is selected from the group consisting of poly(glycolic acid), poly(lactic acid), poly(glycolic acid-lactic acid) and mixtures thereof.

3. Implant according to any of claims 1 and 2, wherein the second polymer component of the matrix is collagen, preferably gelatine.

4. Implant according to any of claims 1 to 3, wherein the primary pores of the matrix have an average pore size diameter of 150 $\mu$m to 300 $\mu$m, preferably 200 $\mu$m to 300 $\mu$m.

5. Implant according to claim 4, wherein the secondary pores of the matrix have an average pore size diameter smaller than the average pore size diameter of the primary pores and being in the range from 50 $\mu$m to 290 $\mu$m, preferably 50 $\mu$m to 150 $\mu$m, more preferably 50 $\mu$m to 100 $\mu$m.

6. Implant according to any of claims 1 to 5, the matrix comprising a porous base layer and a porous cell-embedding layer, the average pore size of the pores in the base layer being smaller than the one of the pores in the cell-embedding layer.

7. Implant according to one of claims 1 to 6, wherein the pores in the base layer of the matrix have an average pore size diameter of 10 $\mu$m to 50 $\mu$m.

8. Implant according to one of claims 1 to 7, wherein the matrix has a porosity of at least 80%, preferably of at least 90%, most preferably between 95% and 99.5%.

9. Implant according to any of claims 1 to 8, further comprising hepatocytes.

10. Implant according to claim 9, wherein the ratio of hepatocytes to cells of Islets of Langerhans is $1 \times 10^6$ hepatocytes to 20'000-500'000 cells of Islets of Langerhans, preferably $1 \times 10^6$ hepatocytes to 20'000-100'000 cells of Islets of Langerhans, most preferably $1 \times 10^6$ hepatocytes to 20'000-70'000 cells of Islets of Langerhans.

11. Implant according to any one of claims 9 or 10 for use in the treatment of chronic liver disease or pancreatic disease and/or chronic liver failure and pancreatic failure.

12. Method for preparing an implant according to one of claims 1 to 11, comprising the steps of

    a. providing a porous matrix according to any of claims 1 to 8,
    b. loading hepatocytes and at least one cell of Islets of Langerhans onto at least one side the matrix, and
    c. folding the matrix with the cells thereon to obtain a bi- or multi-layered structure.

13. Method according to claim 12, wherein step b) involves the loading of cells onto both sides of the matrix.

**Patentansprüche**

1. Implantat zur Gewebezüchtung, umfassend
mindestens eine poröse Matrix zur Gewebezüchtung, wobei die Matrix eine erste biologisch abbaubare und biokompatible Polymerkomponente umfasst, die eine dreidimensionale Primärstruktur mit primären Poren bildet, und ferner eine zweite biologisch abbaubare und biokompatible Polymerkomponente umfasst, die von der ersten Polymerkomponente verschieden ist, die aus der Gruppe ausgewählt ist, bestehend aus Kollagenen, Laminin, Fibronektin und Mischungen davon, wobei die zweite Polymerkomponente eine dreidimensionale Sekundärstruktur mit sekundären Poren formt, wobei die Sekundärstruktur im Innenraum von mindestens einem Teil der primären Poren enthalten ist; und
mindestens eine Langerhans'sche Inselzellen,
wobei die Matrix im Wesentlichen in Form eines Blattes mit einer Dicke im Bereich von 0,1 bis 1,0 mm vorliegt und auf mindestens einer Seite mit Zellen versehen ist, und

wobei das Implantat eine zwei- oder mehrschichtige Struktur aufweist, die durch Falten der Matrix oder durch Übereinanderlegen von zwei oder mehreren Matrizen bereitgestellt wird.

**2.** Implantat nach Anspruch 1, wobei die erste Polymerkomponente der Matrix ausgewählt ist aus der Gruppe bestehend aus Poly(glykolsäure), Poly(milchsäure), Poly(glykolsäure-milchsäure) und Mischungen davon.

**3.** Implantat nach einem der Ansprüche 1 und 2, wobei die zweite Polymerkomponente der Matrix Kollagen, vorzugsweise Gelatine, ist.

**4.** Implantat nach einem der Ansprüche 1 bis 3, wobei die primären Poren der Matrix einen durchschnittlichen Porendurchmesser von 150 $\mu$m bis 300 $\mu$m, vorzugsweise 200 $\mu$m bis 300 $\mu$m aufweisen.

**5.** Implantat nach Anspruch 4, wobei die sekundären Poren der Matrix einen durchschnittlichen Porendurchmesser aufweisen, der kleiner ist als der durchschnittliche Porendurchmesser der primären Poren und im Bereich von 50 $\mu$m bis 290 $\mu$m, vorzugsweise 50 $\mu$m bis 150 $\mu$m, vorzugsweise 50 $\mu$m bis 100 $\mu$m liegt.

**6.** Implantat nach einem der Ansprüche 1 bis 5, wobei die Matrix eine poröse Basisschicht und eine poröse Zelleinbettungsschicht umfasst, wobei die durchschnittliche Porengröße der Poren in der Basisschicht kleiner ist als die der Poren in der Zelleinbettungsschicht.

**7.** Implantat nach einem der Ansprüche 1 bis 6, wobei die Poren in der Basisschicht der Matrix einen durchschnittlichen Porendurchmesser von 10 $\mu$m bis 50 $\mu$m aufweisen.

**8.** Implantat nach einem der Ansprüche 1 bis 7, wobei die Matrix eine Porosität von mindestens 80%, vorzugsweise von mindestens 90%, am bevorzugtesten zwischen 95% und 99,5% aufweist.

**9.** Implantat nach einem der Ansprüche 1 bis 8, des weiteren umfassend Hepatozyten.

**10.** Implantat nach Anspruch 9, wobei das Verhältnis von Hepatozyten zu Langerhans'sehe Inselzellen $1 \times 10^6$ Hepatozyten zu 20'000-500'000 Langerhans'sche Inselzellen, vorzugsweise $1 \times 10^6$ Hepatozyten zu 20'000-100'000 Langerhans'sehe Inselzellen, besonders bevorzugt $1 \times 10^6$ Hepatozyten zu 20'000-70'000 Langerhans'sehe Inselzellen, beträgt.

**11.** Implantat nach einem der Ansprüche 9 oder 10 zur Verwendung bei der Behandlung von chronischer Lebererkrankung oder Pankreaserkrankung und/oder chronischer Leberversagen und Pankreasversagen.

**12.** Verfahren zur Herstellung eines Implantats nach einem der Ansprüche 1 bis 11, umfassend die Schritte von

    a. Bereitstellen einer porösen Matrix nach einem der Ansprüche 1 bis 8,
    b. Laden von Hepatozyten und mindestens einer Langerhans'sehe Inselzellen auf mindestens eine Seite der Matrix, und
    c. Falten der Matrix mit den Zellen darauf, um eine zwei- oder mehrschichtige Struktur zu erhalten.

**13.** Verfahren nach Anspruch 12, wobei Schritt b) das Laden von Zellen auf beide Seiten der Matrix beinhaltet.

**Revendications**

**1.** Implant pour le génie tissulaire, comprenant

- au moins une matrice poreuse pour le génie tissulaire, la matrice comprenant un premier composant polymère biodégradable et biocompatible formant une structure primaire tridimensionnelle avec des pores primaires, et en outre comprenant un second composant polymère biodégradable et biocompatible autre que le premier composant polymère sélectionné dans le groupe consistant en collagènes, laminine, fibronectine et des mélanges de ceux-ci, le second composant polymère formant une structure secondaire tridimensionnelle avec des pores secondaires, la structure secondaire étant contenue dans l'espace intérieur de au moins une partie des pores primaires ; et

- au moins une cellule d'îlots de Langerhans,

la matrice étant essentiellement de la forme d'une feuille avec une épaisseur dans un intervalle de 0.1 à 1.0 mm et étant pourvue de cellules sur au moins une face, et

l'implant présentant une structure bi- ou multicouches fournie en pliant la matrice ou en posant deux ou plus matrices l'une sur l'autre.

2. Implant selon la revendication 1, le premier composant polymère de la matrice étant sélectionné dans le groupe consistant en poly(acide glycolique), poly(acide lactique), poly(acide glycolique - acide lactique) et des mélanges de ceux-ci.

3. Implant selon l'une quelconque des revendications 1 et 2, le second composant polymère de la matrice étant du collagène, préférablement de la gélatine.

4. Implant selon l'une quelconque des revendications 1 à 3, les pores primaires de la matrice présentant un diamètre de taille de pore moyen de 150 $\mu$m à 300 $\mu$m, préférablement de 200 $\mu$m à 300 $\mu$m.

5. Implant selon la revendication 4, les pores secondaires de la matrice présentant un diamètre de taille de pore moyen plus petit que le diamètre de taille de pore moyen des pores primaires et étant dans un intervalle de 50 $\mu$m à 290 $\mu$m, préférablement de 50 $\mu$m à 150 $\mu$m, de façon plus préférée de 50 $\mu$m à 100 $\mu$m.

6. Implant selon l'une quelconque des revendications 1 à 5, la matrice comprenant une couche de base poreuse et une couche d'incorporation de cellules poreuse, la taille moyenne des pores dans la couche de base étant plus petite que celle des pores dans la couche d'incorporation de cellules.

7. Implant selon l'une des revendications 1 à 6, les pores dans la couche de base de la matrice présentant un diamètre de taille de pore moyen de 10 $\mu$m à 50 $\mu$m.

8. Implant selon l'une des revendications 1 à 7, la matrice présentant une porosité d'au moins 80%, préférablement d'au moins 90%, plus préférablement entre 95% et 99.5%.

9. Implant selon l'une quelconque des revendications 1 à 8, comprenant en outre des hépatocytes.

10. Implant selon la revendication 9, le rapport des hépatocytes aux cellules d'îlots de Langerhans étant de $1 \times 10^6$ hépatocytes pour 20'000-500'000 cellules d'îlots de Langerhans, préférablement $1 \times 10^6$ hépatocytes à 20'000-100'000 cellules d'îlots de Langerhans, de façon la plus préférée $1 \times 10^6$ hépatocytes to 20'000-70'000 cellules d'îlots de Langerhans.

11. Implant selon l'une quelconque des revendications 9 à 10 pour l'utilisation dans le traitement d'une maladie chronique du foie ou d'une maladie pancréatique et/ou d'une insuffisance chronique du foie et d'une insuffisance pancréatique.

12. Méthode pour la préparation d'un implant selon l'une des revendications 1 à 11, comprenant les étapes

   a. Fournir une matrice poreuse selon l'une quelconque des revendications 1 à 8,
   b. Charger des hépatocytes et au moins une cellule d'îlots de Langerhans sur au moins un côté de la matrice, et
   c. Plier la matrice avec les cellules dessus pour obtenir une structure bi- ou multicouches.

13. Méthode selon la revendication 12, l'étape b) implique de charger des cellules sur les deux côtés de la matrice.

**EP 3 010 556 B1**

**Patent documents cited in the description**

- EP 2256155 A

- EP 2342317 B1

**Non-patent literature cited in the description**

- **G. CHEN et al.** A biodegradable hybrid sponge nested with collagen microsponges. *Journal of Biomedical Materials Research,* 01 August 2000, 273-279

- **NAOKI KAWAZOE et al.** cell leakproof PLGA-collagen hybrid scaffold for cartilage tissue engineering. *Biotechnology Process, American Institute of Chemical Engineers,* 01 May 2010, vol. 26 (3), 819-826